Europäisches Patentamt

European Patent Office

Office européen des brevets

(19)

(11) Veröffentlichungsnummer: **0 056 250**
**B1**

(12) **EUROPÄISCHE PATENTSCHRIFT**

(45) Veröffentlichungstag der Patentschrift:
27.12.84

(51) Int. Cl.³: **C 07 C 85/145, C 07 C 87/50**

(21) Anmeldenummer: **82100081.7**

(22) Anmeldetag: **08.01.82**

(54) **Verfahren zur Herstellung von Diphenylbasen.**

(30) Priorität: **12.01.81 DE 3100621**

(43) Veröffentlichungstag der Anmeldung:
**21.07.82 Patentblatt 82/29**

(45) Bekanntmachung des Hinweises auf die Patenterteilung:
**27.12.84 Patentblatt 84/52**

(84) Benannte Vertragsstaaten:
**DE FR GB IT**

(56) Entgegenhaltungen:
**EP - A - 0 045 459**
**DE - B - 1 030 833**
**US - A - 1 633 123**

**CHEMICAL ABSTRACTS, Band 52, Nr. 6. März 25, 1958 Zusammenfassung 4530 d Columbus Ohio (US) Miroslav Vécéra, et al: "Separation and identification of the rearrangement products of hydrazobenzene" CHEM. LISTY 51, 1690-2 (1957)**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt am Main 80 (DE)**

(72) Erfinder: **Bodenbenner, Kurt, Dr.,**
**Herman-Kaiser-Strasse 4, D-6200 Wiesbaden (DE)**
Erfinder: **Durruti-Cubria, Manuel, Dr., Stroofstrasse 4, D-6230 Frankfurt am Main 80 (DE)**
Erfinder: **Woernle, Rolf, Dr.,**
**Martin-Wohmann-Strasse 27, D-6238 Hofheim am Taunus (DE)**

**Beschreibung**

Diphenylbasen, insbesondere die in 3,3'-Stellung durch Chlor, Methyl oder Methoxy substituierten 4,4'-Diaminodiphenyle, sind wichtige Ausgangsmaterialien für Disazoverbindungen, wobei diese Stoffe einerseits direkt als Bis-Diazokomponenten dienen und andererseits auch als Bis-N-acetoacetylverbindungen als Kupplungskomponenten eingesetzt werden.

Diphenylbasen werden üblicherweise durch sogenannte Benzidin-Umlagerung aus den entsprechenden Hydrazoaromaten hergestellt, die ihrerseits durch Reduktion der entsprechenden Nitrobenzole gewonnen werden. Die Benzidin-Umlagerung wird durch Mineralsäuren katalysiert. Die Hydrazoaromaten fallen bei deren Herstellung üblicherweise gelöst in einem aromatischen Lösemittel an.

Die Benzidin-Umlagerung der in einem aromatischen Lösemittel gelösten Hydrazoaromaten erfolgt sodann mit Hilfe wässriger Mineralsäure; sie findet in der wässrigen Phase statt, und das sich abscheidende Salz der Diphenylbase bildet hierbei mit dem aromatischen Lösemittel und der Mineralsäure eine Dispersion. Solch eine Verfahrensweise ist in der Deutschen Auslegeschrift Nr. 1 030 833 beschrieben. Sie hat jedoch den erheblichen Nachteil, dass die Dispersion mit fortschreitender Umsetzung so viskos wird, dass sie weder rührbar noch pumpfähig ist. Bei der weiteren Umsetzung ist also der Stoffaustausch auf die Diffusion angewiesen, was zu langen Reaktionszeiten führt. Diese haben wiederum zur Folge, dass störende Nebenreaktionen, z.B. die Disproportionierung des Hydrazoaromaten, stärker hervortreten. Man hat deshalb auch in der Deutschen Auslegeschrift 1 030 833 Massnahmen getroffen, geringer viskose Dispersionen zu erhalten, indem man die Menge an Wasser und Säure sehr hoch hielt. Dies führt allerdings zu den erheblichen Nachteilen, dass die Mengen an Abwasser sehr gross sind, deren Aufarbeitung aufwendig ist und eine kontinuierliche Arbeitsweise technisch mit Vorteil nicht mehr durchgeführt werden kann.

Noch ungünstiger verläuft die Umlagerung, wenn man die Säure wasserfrei der Lösung des Hydrazoaromaten in einem aromatischen Lösemittel zusetzt. Es kommt dann nicht nur zur Disproportionierung, sondern auch zur Bildung unerwünschter Nebenprodukte, z.B. von Diphenylinen, o,o'-Benzidinen und Semidinen. Bei der Umlagerung mit Chlorwasserstoff erhält man z.B. bei Einsatz einer Lösung von 2,2'-Dichlorhydrazobenzol in Toluol nur eine Ausbeute von ca. 30% d.Th. an 3,3'-Dichlorbenzidin.

Es wurde deshalb mit der Europäischen Patentanmeldungs-Veröffentlichung Nr. 0 045 459 A1 (entsprechend der älteren Europäischen Patentanmeldung Nr. 81 105 854.4) vorgeschlagen, zur Durchführung der Benzidin-Umlagerung in kontinuierlicher Arbeitsweise die Mineralsäure in einem so grossen Überschuss einzusetzen, dass eine förderbare Suspension entsteht. Aber auch diese Verfahrensweise hat sich technisch nicht durchsetzen lassen, da grosse Abwassermengen anfallen.

Es blieb deshalb weiterhin die Aufgabe, ein Verfahren dieser Art zu finden, das insbesondere im Hinblick auf eine kontinuierliche Arbeitsweise die anfallende Suspension in rührfähiger und förderbarer, wie (ab)pumpbarer, Form entstehen lässt. Denn es musste bei den heutigen Anforderungen möglich sein, die im Reaktionsansatz verwendeten Produkte, einschliesslich des organischen Lösemittels, wie Benzol, ohne Gefährdung der Gesundheit des Bedienungspersonals leicht handhaben zu können. Dies ist jedoch nicht möglich, wenn gemäss dem Stand der Technik der Reaktionsansatz in pastöser Masse erhalten wird, der nur mit Mühe und unvollständig aus den Reaktionsgefässen entfernt werden kann und mit dem eine kontinuierliche Arbeitsweise nicht möglich ist. Es mussten deshalb Verfahrensmerkmale gefunden werden, die es erlauben, den Reaktionsansatz auch am Ende der Benzidin-Umlagerung in einem Zustand zu erhalten, in welchem der gesamte Ansatz auch an den Rändern der Reaktionsgefässe leicht rührfähig bleibt, er sich leicht pumpen und fördern lässt oder dass man, wie in der diskontinuierlichen Arbeitsweise, diesen Reaktionsansatz aus den Reaktionskesseln leicht und vollständig abpumpen kann.

Mit der vorliegenden Erfindung wurde nunmehr solch ein vorteilhaftes Verfahren zur Herstellung von salzsauren Salzen von Diphenylbasen durch Umlagerung der entsprechenden Hydrazoaromaten in nicht-wassermischbaren aromatischen Lösemitteln mittels Salzsäure gefunden, das dadurch gekennzeichnet ist, dass man in die Lösung der Hydrazoverbindung in dem aromatischen Lösemittel gasförmigen Chlorwasserstoff zusammen mit Wasserdampf und einem inerten Gas, wie beispielsweise Stickstoff, einleitet.

Das Inergas kann vorteilhaft nach der Reaktion erneut in den Prozess zurückgeführt werden.

Als Lösemittel für die Hydrazoverbindung kommen alle inerten aromatischen Stoffe in Betracht, die ein ausreichendes Lösevermögen zeigen. Von technischer Bedeutung sind Toluol, Xylol und Lösemittelgemische, z.B. das unter der Bezeichnung «Solvent-naphta» handelsübliche Gemisch aus m-Xylol und Ethylbenzol. Vorteilhaft verwendet man Lösungen des Hydrazoaromaten, wie sie bei der Reduktion der entsprechenden Nitroverbindungen in einem geeigneten, nicht-wassermischbaren, aromatischen Lösemittel anfallen, insbesondere in einem der genannten Kohlenwasserstoffe.

Der Chlorwasserstoff wird zweckmässig in der einfachen bis zehnfachen stöchiometrischen, zur Salzbildung erforderlichen Menge eingesetzt. Das Verhältnis von Wasser zu Mineralsäure hängt von der Beschaffenheit der sich bildenden Suspension ab. Im allgemeinen liegt es bei 0,5 bis 2,5 Mol, insbesondere 0,5 bis 1,5 Mol Wasser pro Mol Säure. Das Wasser kann mit der Säure gemischt über einen Verdampfer in die Reaktionsmischung eingegeben werden.

Die Reaktionstemperatur richtet sich nach der Reaktivität der Hydrazoverbindung und liegt üblicherweise im Bereich von etwa 0 bis 50°C.

Das Verfahren kann diskontinuierlich wie kontinuierlich durchgeführt werden, so in verschiedenen Apparaten, wie Rührkesseln, Blasensäulen und Umlaufreaktoren. Bei kontinuierlicher Fahrweise ist eine Schaltung als Kaskade möglich.

In den folgenden Beispielen beziehen sich die Prozentangaben auf das Gewicht, wenn nicht anders angegeben.

*Beispiel 1*

Eine Apparatur aus einem Vierhalskolben, versehen mit Turbinenrührer, Rückflusskühler, Gaseinleitungsrohr und Thermometer, wird über das Gaseinleitungsrohr an einen elektrisch beheizten Kolben angeschlossen, in den mit einer Dosierpumpe Wasser eingepumpt und verdampft werden kann. In diesen Kolben wird ausserdem ein konstanter Gasstrom eingeleitet, der aus einer Mischung aus Chlorwasserstoff und Stickstoff besteht. Das verdampfte Wasser wird von diesem Gasstrom in den Vierhalskolben transportiert.

In dem Vierhalskolben wird eine Lösung von 25 g 2,2'-Dichlorhydrazobenzol in 250 g Toluol vorgelegt. In den elektrisch beheizten Kolben werden 12 l/h HCl und 160 l/h N$_2$ eingeleitet, gleichzeitig werden 30 g/h H$_2$O eingepumpt. Das Gasgemisch gelangt in den Vierhalskolben, wo die Umlagerung unter Bildung einer Suspension eintritt. Dabei wird die Temperatur bei 25 bis 30°C gehalten.

Nach einer Reaktionszeit von ca. 1 h wird die Suspension des gebildeten Dichlorbenzidin-Hydrochlorids mit 25%iger Ammoniaklösung in der Siedehitze neutralisiert. Die organische Phase wird abgetrennt und auf 40 bis 50 g eingeengt. Beim Abkühlen kristallisiert 3,3'-Dichlor-4,4'-diaminodiphenyl aus, das abgesaugt und zweimal mit je 13 g Toluol gewaschen wird. Es werden 23 g eines Filterkuchens erhalten, der gemäss Titration aus 95% Diphenylbase besteht. Dies entspricht einer Ausbeute von 87,4% d.Th., bezogen auf das eingesetzte Dichlor-hydrazobenzol.

*Beispiel 2*

In einer Blasensäule mit Frittenboden wird eine Lösung aus 10% Dichlorhydrazobenzol in Toluol mit einer Gasmischung aus 6 Vol.-% Chlorwasserstoff, 9 Vol.-% Wasserdampf und 85 Vol.-% Stickstoff bei 1 bar und bei einer Temperatur von 25°C kontinuierlich umgelagert. Die Mengenströme werden so gewählt, dass pro Mol Dichlorhydrazobenzol 4 Mol Chlorwasserstoff und 6 Mol Wasserdampf eingesetzt werden. Der Stickstoff wird im Kreislauf gefahren und vor Wiederverwendung in der Säule mit HCl-Gas und Wasserdampf auf die obigen Verhältnisse aufgestärkt.

Die sich bildende Suspension wird aus dem unteren Teil des Reaktionsapparats kontinuierlich abgezogen. Die Ausbeute ist wie in Beispiel 1.

**Patentansprüche**

1. Verfahren zur Herstellung von salzsauren Salzen von Diphenylbasen durch Umlagerung der entsprechenden Hydrazoaromaten in nicht-wassermischbaren aromatischen Lösemitteln mit Salzsäure, dadurch gekennzeichnet, dass man in die Lösung der Hydrazoverbindung in dem aromatischen Lösemittel gasförmigen Chlorwasserstoff zusammen mit Wasserdampf und einem inerten Gas einleitet.

2. Verfahren nach Anspruch 1, dadurch gekennzeichnet, dass pro Mol Chlorwasserstoff 0,5 bis 2,5 Mol Wasser eingesetzt werden.

3. Verfahren nach Anspruch 1 oder 2, dadurch gekennzeichnet, dass die ein- bis zehnfache der stöchiometrisch zur Salzbildung erforderlichen Menge an Chlorwasserstoff eingesetzt wird.

**Claims**

1. A process for the manufacture of hydrochloric acid salts of diphenyl bases by rearrangement of the corresponding hydrazoaromatic compounds with hydrochloric acid in water-immiscible aromatic solvents, characterized by that gaseous hydrogen chloride is introduced in admixture with steam and an inert gas to the solution of the hydrazo compound in the aromatic solvent.

2. The process according to Claim 1, characterized in that 0.5 to 2.5 mols of water per mol of hydrogen chloride are used.

3. The process according to Claim 1 or 2, characterized in that the 1- to 10-fold stoichiometric amount of hydrogen chloride, required for salt-formation, is introduced.

**Revendications**

1. Procédé de préparation de sels chlorhydriques de base diphénylique par transposition à l'aide d'acide chlorhydrique des composés hydrazo aromatiques correspondants dans des solvants aromatiques non miscibles à l'eau, procédé caractérisé en ce que, dans la solution du composé hydrazoïque dans le solvant aromatique, on itroduit du chlorure d'hydrogène gazeux ainsi que de la vapeur d'eau et un gaz inerte.

2. Procédé selon la revendication 1, caractérisé en ce qu'on utilise par mole de chlorure d'hydrogène 0,5 à 2,5 moles d'eau.

3. Procédé selon la revendication 1 ou 2, caractérisé en ce qu'on utilise une quantité de chlorure d'hydrogène représentant un à dix fois la quantité stoechiométriquement nécessaire pour la formation d'un sel.